# EUROPEAN PATENT APPLICATION

(11) **EP 4 521 403 A1**
(43) Date of publication of application: **12.03.2025**
(21) Application number: 23826194.5
(22) Date of filing: 12.06.2023
(51) Int. Cl.: G10L 25/66, H04R 1/08, H04R 1/10

(54) **COUGH MONITORING METHOD AND RELATED DEVICE**

(30) Priority: 20.06.2022 CN 202210697764
(71) Applicant: Huawei Technologies Co., Ltd., Shenzhen, Guangdong 518129 (CN)
(72) Inventor: WANG, Wei, Shenzhen, Guangdong 518129 (CN); YE, Jilong, Shenzhen, Guangdong 518129 (CN); LI, Jing, Shenzhen, Guangdong 518129 (CN)
(74) Representative: Pfenning, Meinig & Partner mbB
(86) International application number: PCT/CN2023/099674
(87) International publication number: WO 2023/246545

(57) **Abstract**

A cough monitoring method and a related device are disclosed to reduce a risk to user privacy security and power consumption of a device. The method includes: **In** response to a detected vibration signal, a headset activates a microphone to perform audio recording to obtain audio recording data, where the headset is worn around a head and neck region of a user; the headset detects whether the audio recording data includes a cough sound; and if the audio recording data includes the cough sound, the headset sends the audio recording data to a smart terminal device, so that the smart terminal device analyzes and collects statistics on cough information about the user based on the audio recording data.

## Description

This application claims priority to Chinese Patent Application No. 202210697764.5, filed with the China National Intellectual Property Administration on June 20, 2022 and entitled "COUGH MONITORING METHOD AND RELATED DEVICE", which is incorporated herein by reference in its entirety.

### TECHNICAL FIELD

This application relates to the field of internet technologies, and in particular, to a cough monitoring method and a related device.

### BACKGROUND

A cough is the most common symptom in respiratory specialist outpatients and community outpatients. A cough is usually caused by inflammation of tracheae and bronchi, foreign bodies, or physical or chemical irritation. A cough plays a protective role in clearing foreign bodies and secretions from a respiratory tract, but a frequent and intense cough severely affects a patient's work, study, and quality of life.

Causes of a cough are complex. However, with development of medicine in recent years, scholars at home and abroad have a deeper understanding of the cough. Coughs caused by different diseases present different cough manifestations. Usually, patients who cough mainly at night are first considered for a diagnosis of cough variant asthma. Coughs that occur in winter and spring are considered for a diagnosis of chronic bronchitis. In clinical diagnosis, doctors focus on information such as a time, a frequency, and duration of the cough, and assess a disease type and a severity. Therefore, continuous monitoring of the cough is of great value to disease diagnosis and assessment.

Currently, a cough monitor is usually used to continuously monitor the cough. The cough monitor includes a microphone and a memory. The microphone of the cough monitor works on a 24-hour basis to record audio data. However, with the microphone constantly working, it poses a risk to user privacy security, and leads to high power consumption of the device, and high storage resource requirements.

### SUMMARY

This application provides a cough monitoring method and a related device to reduce a risk to user privacy security and power consumption of a device.

According to a first aspect, a cough monitoring method is provided. The method includes: In response to a detected vibration signal, a headset activates a microphone to perform audio recording to obtain audio recording data, where the headset is worn around a head and neck region of a user; the headset detects whether the audio recording data includes a cough sound; and if the audio recording data includes the cough sound, the headset sends the audio recording data to a smart terminal device, so that the smart terminal device analyzes and collects statistics on cough information about the user based on the audio recording data. When the headset worn around the head and neck region of the user detects the vibration signal, the microphone is activated to perform audio recording, so that the microphone does not need to constantly enable audio recording. This can reduce a risk to user privacy security, and reduce power consumption of the headset and a storage resource requirement.

In a possible implementation, that the headset detects whether the audio recording data includes a cough sound includes: The headset compares a similarity between the audio recording data and cough sound data, where the cough sound data is previously recorded cough sound data of the user; and if the similarity between the audio recording data and the cough sound data is greater than or equal to a threshold, the headset determines that the audio recording data includes the cough sound; or if the similarity between the audio recording data and the cough sound data is less than the threshold, the headset determines that the audio recording data does not include the cough sound. Matching the previously recorded cough sound data of the user with the audio recording data can improve accuracy of recognizing the cough sound.

In a possible implementation, the method further includes: The headset records vibration data corresponding to the detected vibration signal; and that the headset sends the audio recording data to a smart terminal device, so that the smart terminal device analyzes and collects statistics on cough information based on the audio recording data includes: the headset sends the audio recording data and the vibration data to the smart terminal device, so that the smart terminal device analyzes and collects the statistics on the cough information based on the audio recording data and the vibration data. The statistics on the cough information are collected with reference to the vibration data corresponding to the vibration signal, so that accuracy of cough statistics information can be improved.

In a possible implementation, the audio recording data includes audio data and an acquisition time of the audio data, and the cough information includes at least one of a cough time, a quantity of coughs, a cough frequency, a cough trend, and cough duration.

In a possible implementation, the method further includes: If the audio recording data does not include the cough sound, the headset deletes the audio recording data. In this way, storage space is reduced, and the storage resource requirement is reduced.

According to a second aspect, a cough monitoring method is provided. The method includes: A headset detects a vibration signal, where the headset is worn around a head and neck region of a user; and in response to the vibration signal, the headset sends an indication message to a smart terminal device, so that the smart terminal device activates, based on the indication message, a microphone to perform audio recording to obtain audio recording data, and analyzes and collects statistics on cough information about the user based on the audio recording data when it is determined that the audio recording data includes a cough sound. When the headset detects the vibration signal, the indication message for indicating audio recording is sent to the smart terminal device, so that the microphone does not need to constantly enable audio recording. This can reduce a risk to user privacy security, power consumption, and storage resource consumption. In addition, the smart terminal device performs audio recording and detects the audio recording data, so that requirements for a computing capability and a storage capability of the headset can be reduced, and costs of the headset can be reduced.

In a possible implementation, the audio recording data includes audio data and an acquisition time of the audio data, and the cough information includes at least one of a cough time, a quantity of coughs, a cough frequency, a cough trend, and cough duration.

According to a third aspect, a cough monitoring method is provided. The method includes: A smart terminal device receives an indication message sent by a headset, where the headset is worn around a head and neck region of a user, and the indication message is generated when the headset detects a vibration signal; in response to the indication message, the smart terminal device activates a microphone to perform audio recording to obtain audio recording data; the smart terminal device detects and determines that the audio recording data includes a cough sound; and the smart terminal device analyzes and collects statistics on cough information about the user based on the audio recording data.

In a possible implementation, that the smart terminal device detects and determines that the audio recording data includes a cough sound includes: The smart terminal device compares a similarity between the audio recording data and cough sound data, where the cough sound data is previously recorded cough sound data of the user; and when the smart terminal device determines that the similarity between the audio recording data and the cough sound data is greater than or equal to a threshold, the smart terminal device determines that the audio recording data includes the cough sound.

In a possible implementation, the method further includes: The smart terminal device receives vibration data sent by the headset, where the vibration data is data that corresponds to the detected vibration signal and that is recorded by the headset; and that the smart terminal device analyzes and collects statistics on cough information based on the audio recording data includes: the smart terminal device analyzes and collects the statistics on the cough information based on the audio recording data and the vibration data.

In a possible implementation, the audio recording data includes audio data and an acquisition time of the audio data, and the cough information includes at least one of a cough time, a quantity of coughs, a cough frequency, a cough trend, and cough duration.

According to a fourth aspect, a cough monitoring method is provided. The method includes: A headset detects a vibration signal, where the headset is worn around a head and neck region of a user; in response to the vibration signal, the headset sends an indication message to a smart terminal device; in response to the indication message, the smart terminal device activates a microphone to perform audio recording to obtain audio recording data; the smart terminal device detects and determines that the audio recording data includes a cough sound; and the smart terminal device analyzes and collects statistics on cough information about the user based on the audio recording data.

In a possible implementation, that the smart terminal device detects and determines that the audio recording data includes a cough sound includes: The smart terminal device compares a similarity between the audio recording data and cough sound data, where the cough sound data is previously recorded cough sound data of the user; and when the smart terminal device determines that the similarity between the audio recording data and the cough sound data is greater than or equal to a threshold, the smart terminal device determines that the audio recording data includes the cough sound.

In a possible implementation, the method further includes: The smart terminal device receives vibration data sent by the headset, where the vibration data is data that corresponds to the detected vibration signal and that is recorded by the headset; and that the smart terminal device analyzes and collects statistics on cough information based on the audio recording data includes: the smart terminal device analyzes and collects the statistics on the cough information based on the audio recording data and the vibration data.

In a possible implementation, the audio recording data includes audio data and an acquisition time of the audio data, and the cough information includes at least one of a cough time, a quantity of coughs, a cough frequency, a cough trend, and cough duration.

In a possible implementation, when it is determined that the audio recording data does not include the cough sound, the smart terminal device deletes the audio recording data.

According to a fifth aspect, a cough monitoring system is provided. The system includes a headset and a smart terminal device. The headset is worn around a head and neck region of a user, and is configured to detect a vibration signal. The headset is further configured to: when detecting the vibration signal, activate a microphone to perform audio recording to obtain audio recording data. The headset is further configured to detect whether the audio recording data includes a cough sound. The headset is further configured to send the audio recording data to the smart terminal device when the audio recording data includes the cough sound. The smart terminal device is configured to analyze and collect statistics on cough information about the user based on the audio recording data.

In a possible implementation, the headset is specifically configured to compare a similarity between the audio recording data and cough sound data, where the cough sound data is previously recorded cough sound data of the user; and if the similarity between the audio recording data and the cough sound data is greater than or equal to a threshold, the headset determines that the audio recording data includes the cough sound; or if the similarity between the audio recording data and the cough sound data is less than the threshold, the headset determines that the audio recording data does not include the cough sound.

In a possible implementation, the headset is further configured to record vibration data corresponding to the detected vibration signal. The headset is further configured to send the audio recording data to the smart terminal device, so that the smart terminal device analyzes and collects the statistics on the cough information based on the audio recording data and the vibration data.

In a possible implementation, the audio recording data includes audio data and an acquisition time of the audio data, and the cough information includes at least one of a cough time, a quantity of coughs, a cough frequency, a cough trend, and cough duration.

In a possible implementation, the headset is further configured to delete the audio recording data when the audio recording data does not include the cough sound.

According to a sixth aspect, a cough monitoring system is provided. The system includes a headset and a smart terminal device.

The headset is worn around a head and neck region of a user, and is configured to detect a vibration signal.

The headset is configured to send an indication message to the smart terminal device when the vibration signal is detected.

The smart terminal device is configured to activate, based on the indication message, a microphone to perform audio recording to obtain audio recording data.

The smart terminal device is further configured to analyze and collect statistics on cough information about the user based on the audio recording data when it is detected and determined that the audio recording data includes a cough sound.

In a possible implementation, the smart terminal device is specifically configured to: compare a similarity between the audio recording data and cough sound data, where the cough sound data is previously recorded cough sound data of the user; and when it is determined that the similarity between the audio recording data and the cough sound data is greater than or equal to a threshold, determine that the audio recording data includes the cough sound.

In a possible implementation, the smart terminal device is further configured to receive vibration data sent by the headset, where the vibration data is data that corresponds to the detected vibration signal and that is recorded by the headset; and that the smart terminal device analyzes and collects statistics on cough information based on the audio recording data includes: the smart terminal device analyzes and collects the statistics on the cough information based on the audio recording data and the vibration data.

In a possible implementation, the audio recording data includes audio data and an acquisition time of the audio data, and the cough information includes at least one of a cough time, a quantity of coughs, a cough frequency, a cough trend, and cough duration.

In a possible implementation, when it is determined that the audio recording data does not include the cough sound, the smart terminal device deletes the audio recording data.

According to a seventh aspect, a headset is provided. The headset includes: a vibration detection module, configured to detect a vibration signal; an audio recording module, configured to: when the vibration detection module detects the vibration signal, activate a microphone to perform audio recording to obtain audio recording data; a cough sound detection module, configured to detect whether the audio recording data includes a cough sound; and a sending module, configured to send the audio recording data to a smart terminal device when it is determined that the audio recording data includes the cough sound, so that the smart terminal device analyzes and collects statistics on cough information about a user based on the audio recording data.

In a possible implementation, the cough sound detection module is specifically configured to: compare a similarity between the audio recording data and cough sound data, where the cough sound data is previously recorded cough sound data of the user; and when the similarity between the audio recording data and the cough sound data is greater than or equal to a threshold, determine that the audio recording data includes the cough sound; or when the similarity between the audio recording data and the cough sound data is less than the threshold, determine that the audio recording data does not include the cough sound.

In a possible implementation, the headset further includes: a recording module, configured to record vibration data corresponding to the detected vibration signal. The sending module is further configured to send the vibration data to the smart terminal device, so that the smart terminal device analyzes and collects the statistics on the cough information based on the audio recording data and the vibration data.

In a possible implementation, the audio recording data includes audio data and an acquisition time of the audio data, and the cough information includes at least one of a cough time, a quantity of coughs, a cough frequency, a cough trend, and cough duration.

According to an eighth aspect, a headset is provided. The headset includes: a vibration detection module, configured to detect a vibration signal; and a sending module, configured to send an indication message to a smart terminal device when the vibration signal is detected, so that the smart terminal device activates, based on the indication message, a microphone to perform audio recording to obtain audio recording data, and analyzes and collects statistics on cough information about a user based on the audio recording data when it is determined that the audio recording data includes a cough sound.

According to a ninth aspect, a cough monitoring apparatus is provided. The apparatus includes: a receiving module, configured to receive an indication message sent by a headset, where the headset is worn around a head and neck region of a user, and the indication message is generated when the headset detects a vibration signal; an audio recording module, configured to: when the indication message is received, activate a microphone to perform audio recording to obtain audio recording data; a detection module, configured to detect and determine that the audio recording data includes a cough sound; and a statistics analysis and collection module, configured to analyze and collect statistics on cough information about the user based on the audio recording data.

In a possible implementation, the detection module is specifically configured to: compare a similarity between the audio recording data and cough sound data, where the cough sound data is previously recorded cough sound data of the user; and when it is determined that the similarity between the audio recording data and the cough sound data is greater than or equal to a threshold, determine that the audio recording data includes the cough sound.

In a possible implementation, the receiving module is further configured to receive vibration data sent by the headset, where the vibration data is data that corresponds to the detected vibration signal and that is recorded by the headset. The statistics analysis and collection module is configured to analyze and collect the statistics on the cough information based on the audio recording data and the vibration data.

In a possible implementation, the apparatus includes: a deletion module, configured to delete the audio recording data when the audio recording data does not include the cough sound.

According to a tenth aspect, a cough monitoring device is provided. The device includes a processor and a memory. The processor is coupled to the memory, and the processor is configured to perform, based on instructions stored in the memory, the cough monitoring method in any one of the first aspect to the third aspect or any possible implementation of the first aspect to the third aspect.

According to an eleventh aspect, a computer-readable storage medium is provided. The computer-readable storage medium includes instructions. When the computer-readable storage medium is run on a computer, the computer is enabled to perform the cough monitoring method in any one of the first aspect to the third aspect or any possible implementation of the first aspect to the third aspect.

### BRIEF DESCRIPTION OF DRAWINGS

FIG. 1 is a diagram of a structure of an embodiment of a cough monitoring system according to this application;
FIG. 2 is a schematic flowchart of a first embodiment of a cough monitoring method according to this application;
FIG. 3 is a schematic flowchart of a second embodiment of a cough monitoring method according to this application;
FIG. 4 is a schematic flowchart of a third embodiment of a cough monitoring method according to this application;
FIG. 5 is a schematic flowchart of a fourth embodiment of a cough monitoring method according to this application;
FIG. 6 is a schematic flowchart of a fifth embodiment of a cough monitoring method according to this application;
FIG. 7 is a schematic flowchart of a sixth embodiment of a cough monitoring method according to this application;
FIG. 8 is a diagram of a structure of an embodiment of a headset according to this application;
FIG. 9 is a diagram of a structure of another embodiment of a headset according to this application;
FIG. 10 is a diagram of a structure of an embodiment of a cough monitoring apparatus according to this application; and
FIG. 11 is a diagram of a structure of an embodiment of a cough monitoring device according to this application.

### DESCRIPTION OF EMBODIMENTS

This application provides a cough monitoring method and a related device to reduce a risk to user privacy security and power consumption of a device.

The terms used in this application are merely for the purpose of describing specific embodiments, but are not intended to limit this application. The terms "a", "the", and "this" of singular forms used in this application and the appended claims are also intended to include plural forms, unless otherwise specified in a context clearly. It should also be understood that the term "and/or" used in this specification indicates and includes any or all possible combinations of one or more associated listed items. The character "/" in this specification generally indicates an "or" relationship between associated objects.

It should be understood that, although terms such as first, second, and third may be used in this application to describe various packets/frames, requests, and terminal devices, these packets/frames, requests, and terminal devices should not be limited to these terms. These terms are merely used to distinguish between packets/frames, requests, and terminal devices. For example, without departing from the scope of this application, a first terminal device may also be referred to as a second terminal device. Similarly, a second terminal device may also be referred to as a first terminal device.

Depending on the context, for example, words "if" used herein may be explained as "while" or "when" or "in response to determining" or "in response to detecting". Similarly, depending on the context, phrases "if determining" or "if detecting (a stated condition or event)" may be explained as "when determining" or "in response to determining" or "when detecting (the stated condition or event)" or "in response to detecting (the stated condition or event)".

In this application, "when" is not strictly limited to a meaning of "at the same time" and further has a meaning of "after".

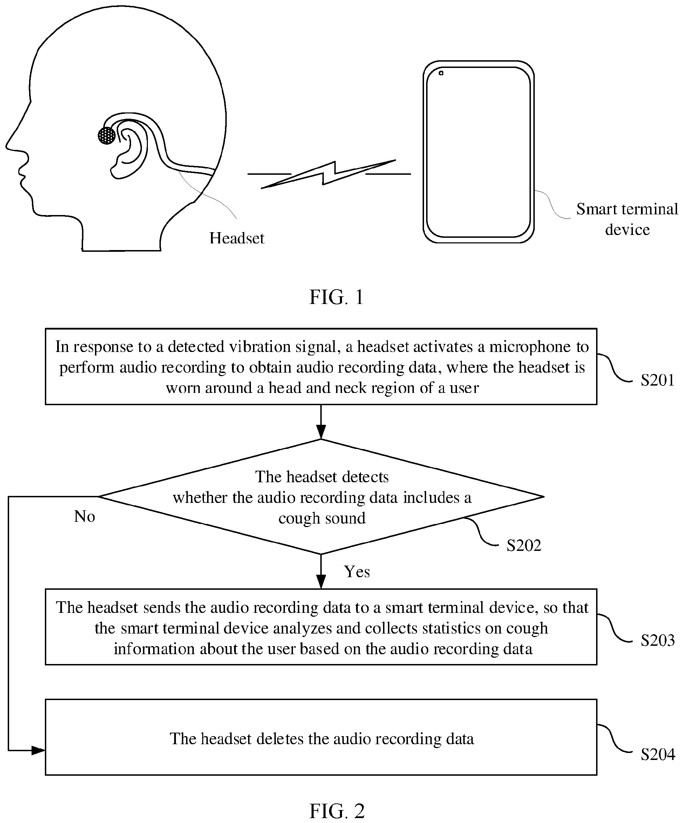

FIG. 1 is a diagram of a structure of an embodiment of a cough monitoring system according to this application. The cough monitoring system in this embodiment includes a headset and a smart terminal device. In this embodiment, the headset has a vibration detection function. The headset specifically includes, for example, a vibration sensor (not shown in the figure), configured to detect a vibration signal. The smart terminal device is configured to analyze cough information.

The headset is worn around a head and neck region of a user. When the user coughs, a vocal cord and a head may vibrate, so that the headset worn around a head region or a neck region of the user can accurately detect the vibration signal when the user coughs.

The headset may be a bone conduction headset. The bone conduction headset is generally worn around the head region of the user and is close to a skull of the user. Skull vibration generated when the user coughs can be accurately detected by a vibration sensor of the bone conduction headset. Alternatively, the headset may be a neck-mounted headset, and the vibration sensor may be disposed on a neck-mounted part of the headset, so that the vibration sensor is close to the vocal cord of the user. Vocal cord vibration generated when the user coughs can be accurately detected by the vibration sensor of the neck-mounted part.

The smart terminal device may be a terminal device with computing power, such as a smartphone or a tablet computer.

A communication connection is established between the headset and the smart terminal device, and the headset and the smart terminal device may exchange data based on the communication connection. In some implementations, the headset and the smart terminal device may be connected via Bluetooth. In some other implementations, the headset and the smart terminal device may alternatively be connected via a wireless local area network (wireless local area network, WLAN), the smart terminal device serves as a wireless hotspot, and the headset accesses to the wireless hotspot to connect to the smart terminal device. In some other implementations, the headset and the smart terminal device may alternatively be connected via near field communication (near field communication, NFC).

After detecting the vibration signal, the headset may activate a microphone of the headset to perform audio recording to obtain audio recording data. The headset detects whether the audio recording data includes a cough sound. Alternatively, after detecting the vibration signal, the headset may inform the smart terminal device to activate a microphone of the smart terminal device to perform audio recording to obtain audio recording data. The smart terminal device detects whether the audio recording data includes a cough sound. Certainly, in some other implementations, alternatively, after the headset detects the vibration signal, the headset may perform audio recording to obtain audio recording data. The headset sends the audio recording data to the smart terminal device, and the smart terminal device detects whether the audio recording data includes a cough sound.

The following separately describes a process of performing audio recording and detecting a cough sound by the headset, and a process of performing audio recording and detecting a cough sound by the smart terminal device.

FIG. 2 is a schematic flowchart of a first embodiment of a cough monitoring method according to this application. This embodiment includes the following steps.

S201: In response to a detected vibration signal, a headset activates a microphone to perform audio recording to obtain audio recording data, where the headset is worn around a head and neck region of a user.

After a vibration sensor of the headset detects the vibration signal, the headset activates the microphone of the headset for audio recording.

The vibration sensor generates mechanical vibration under impact of vibration of an external environment, and converts a mechanical vibration amount into an electrical signal, to detect the vibration signal. The vibration sensor is passively triggered to detect the vibration signal, and can remain in a low-power consumption state when the vibration sensor is not triggered. The microphone is activated to perform audio recording only after the headset detects the vibration signal, and the microphone does not need to continuously enable audio recording. This not only reduces power consumption of the headset, but also protects user privacy.

A time at which the headset ends audio recording may be determined based on the vibration signal. For example, if the headset detects no vibration signal in a preset time period after detecting a vibration signal last time, the headset may stop audio recording, to obtain audio recording data. The preset time period may be, for example, 30 seconds, 1 minute, 2 minutes, or 5 minutes. This is not limited herein.

The audio recording data includes an acquisition time of the audio recording data. The acquisition time specifically includes a start time, an end time, and the like. The acquisition time of the audio recording data is used for analyzing and collecting statistics on cough information.

It may be understood that the headset does not activate the microphone every time the headset detects the vibration signal. For example, if the microphone is already activated when the headset detects the vibration signal, the microphone does not need to be activated again.

S202: The headset detects whether the audio recording data includes a cough sound.

A cough is usually sudden and explosive. An initial stage of the cough is an explosive stage, which features short duration, strong energy, a rapid increase in a sound amplitude, and the like. After the explosive phase, a relatively stable phase is entered. This phase lasts longer than the explosive phase, but the energy is weaker than that of the explosive phase.

Therefore, the cough sound has a characteristic that is different from other speech signals. At least one characteristic parameter that can represent the cough sound may be extracted from the audio recording data, and whether the audio recording data includes the cough sound is recognized based on these characteristic parameters and a corresponding detection algorithm. The characteristic parameter representing the cough sound includes, for example, at least one of a zero-crossing rate, short-term energy, a perceptual linear predictive (perceptual linear predictive, LPL) coefficient, a linear predictive cepstral coefficient (linear predictive cepstral coefficient, LPCC), and a mel-frequency cepstral coefficient (Mel-frequency cepstral coefficient, MFCC).

The detection algorithm may be an algorithm such as a Fisher discriminant algorithm, dynamic time warping, a minimum nearest neighbor algorithm, a hidden Markov model, a Gaussian mixture model, a segment Gaussian model, an artificial neural network, or a support vector machine.

In this embodiment, a function of detecting, by the headset, whether the audio recording data includes the cough sound is to determine whether to continue to store the audio recording data in the headset, or whether to send the audio recording data to a smart terminal device. Specifically, when the headset detects that the audio recording data includes the cough sound, the headset may continue to store the audio recording data until the smart terminal device pulls the audio recording data from the headset; and the audio recording data is sent to the smart terminal device, that is, S203 is performed. Certainly, the headset may alternatively send the audio recording data to the smart terminal device immediately when the headset detects that the audio recording data includes the cough sound. When the headset determines that the audio recording data does not include the cough sound, S204 is performed.

After receiving cough sound data, the smart terminal device further analyzes and collects the statistics on the cough information based on the audio recording data. Therefore, the detection algorithm and the characteristic parameter used by the headset to detect whether the audio recording data includes the cough sound may comprehensively consider a computing capability and accuracy of the headset. A characteristic parameter and a detection algorithm that have a minimum requirement on the computing capability may be selected under a condition of satisfying specified accuracy of detecting the cough sound. For example, a zero-crossing rate may be selected as the characteristic parameter, and a Fisher discriminant algorithm may be selected as the detection algorithm. A detection algorithm and a characteristic parameter used by the headset may be selected according to an actual requirement, and this is not limited herein.

In a possible implementation, to improve accuracy of detecting the cough sound in the audio recording data, the headset may previously capture and store cough sound data of the user, and match the audio recording data with the cough sound data to determine whether the audio recording data includes the cough sound.

Specifically, when the headset is worn around the head and neck region of the user and is connected to the smart terminal device, the microphone of the headset is activated to capture the cough sound data of the user who actively coughs, and stores the cough sound data. Alternatively, the smart terminal device captures the cough sound data of the user who actively coughs, and sends the cough sound data to the headset for storage. The cough sound data may be original audio data, or may be data that is extracted and that corresponds to the characteristic parameter that can represent the cough sound of the user. The cough sound data may include captured cough sounds of the user who actively coughs a plurality of times, or include data corresponding to a characteristic parameter of the captured cough sounds of the user who actively coughs a plurality of times, thereby increasing a quantity of samples and improving accuracy of detecting and recognizing the cough sound.

The headset compares a similarity between the audio recording data and the cough sound data. If the similarity between the audio recording data and the cough sound data is greater than or equal to a threshold, the headset determines that the audio recording data includes the cough sound. If the similarity between the audio recording data and the cough sound data is less than the threshold, the headset determines that the audio recording data does not include the cough sound. The similarity may be indicated by an index such as a cosine similarity between the audio recording data and the cough sound data, a Euclidean distance, or a Manhattan distance.

S203: The headset sends the audio recording data to the smart terminal device, so that the smart terminal device analyzes and collects the statistics on the cough information about the user based on the audio recording data.

The headset may periodically send the acquired audio recording data to the smart terminal device. Alternatively, the headset may immediately send the audio recording data to the smart terminal device after acquiring the audio recording data. In another possible implementation, when a cough monitoring client running on the smart terminal device refreshes data, the smart terminal device may pull the audio recording data from the headset.

Because the smart terminal device has a stronger computing capability and a stronger storage capability, the smart terminal device may more accurately analyze and collect statistics based on the audio recording data to obtain the cough information about the user. The detection algorithm used by the smart terminal device may be a more accurate algorithm, and the characteristic parameter may be a combination of a plurality of characteristic parameters that can more accurately represent the cough sound. For example, the algorithm may be a hidden Markov model, an artificial neural network, a support vector machine, or the like, and the characteristic parameter may include a zero-crossing rate, short-term energy, a mel-frequency cepstral coefficient, and the like. The detection algorithm and the characteristic parameter used by the smart terminal device may be selected according to an actual requirement. This is not limited herein.

The cough information includes at least one of the following: a cough time, a quantity of coughs, a cough frequency, a cough trend, and cough duration. When one cough sound in the audio recording data is detected, the quantity of coughs is increased by 1, and a cough time corresponding to the cough sound is recorded. Based on the quantity of coughs and the cough time, information such as the cough frequency, the cough trend, and the cough duration may be further obtained. The cough frequency is, for example, a sum of the quantity of coughs per minute, 5 minutes, 10 minutes, 30 minutes, 1 hour, 6 hours, 12 hours, or 24 hours. The cough trend may be an increase or decrease trend of a sum of the quantity of coughs in a time period relative to a sum of the quantity of coughs in a previous time period, for example, a change trend of the quantity of coughs every hour. The cough duration may be corresponding duration when cough sounds appear in a concentrated manner, or may be duration from an earliest cough sound to a latest cough sound detected in the audio recording data.

The cough information may be cough data that is of the user and that is obtained by analyzing audio recording data acquired when the user coughs last time, or may be total cough data that is of the user and that is collected through statistics in a preset time period (for example, 6 hours, 12 hours, 24 hours, 2 days, 7 days, the current day, the current week, or the current month).

The cough information obtained by the smart terminal device through statistics analysis and collection may be displayed on the cough monitoring client of the smart terminal device, so that the user can intuitively learn a cough condition and a physical condition.

Optionally, after the headset sends the audio recording data to the smart terminal device, local audio recording data may be deleted to release storage space of the headset.

S204: The headset deletes the audio recording data.

In some cases, vibration of the head and neck region of the user is not caused by a cough. In this case, the audio recording data may not include the cough sound. When there is no cough sound in audio recording data, the audio recording data may be deleted to release storage space of the headset.

In this embodiment, the headset activates the microphone to perform audio recording only after detecting the vibration signal, so that a risk to user privacy security can be reduced, and power consumption of the headset can be further reduced. After detecting that the audio recording data includes the cough sound, the headset sends the audio recording data to the smart terminal device. Otherwise, the audio recording data is deleted. In this way, occupation of a storage resource of the headset can be reduced, and unnecessary data transmission between the headset and the smart terminal device is reduced, thereby reducing power consumption of the headset.

When the headset activates the microphone for audio recording, captured sounds may further include some sounds in an environment in which the user is located. A sound in the environment may cause false detection. For example, an acquired cough sound of another person is recognized as a cough sound of the user, which may affect accuracy of detecting the cough sound.

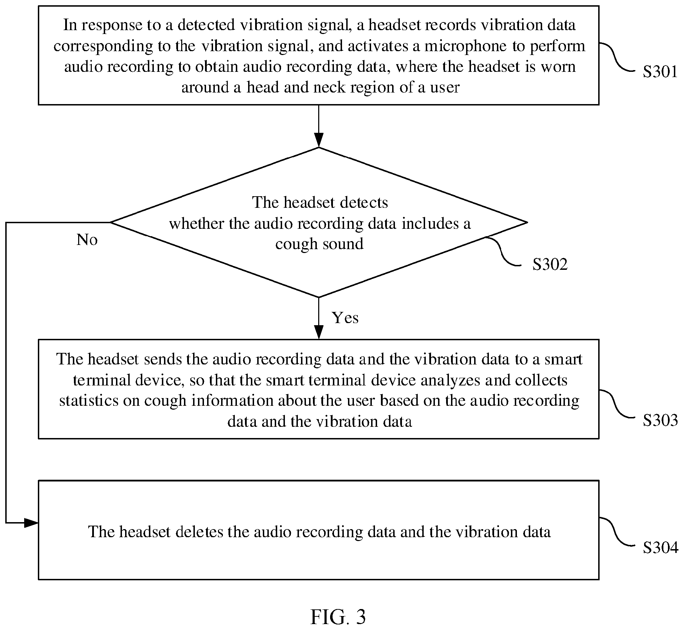

Therefore, in this application, the statistics on the cough information are further analyzed and collected with reference to the vibration data corresponding to vibration signal detected by the headset. FIG. 3 is a schematic flowchart of a second embodiment of a cough monitoring method according to this application. For brevity of description, a similarity between this embodiment and the first embodiment of the cough monitoring method is not described herein again. This embodiment includes the following steps.

S301: In response to a detected vibration signal, a headset records vibration data corresponding to the vibration signal, and activates a microphone to perform audio recording to obtain audio recording data, where the headset is worn around a head and neck region of a user.

The vibration data includes, for example, a time when the vibration signal is detected. The vibration data may further include a vibration characteristic value such as a time domain characteristic value of the vibration signal and/or a frequency domain characteristic value of the vibration signal. The time domain characteristic value includes, for example, an average value, a variance, a kurtosis, or a peak value of the vibration signal. The frequency domain characteristic value includes, for example, a frequency or energy.

Each time when the headset detects a vibration signal, an acquisition time of the vibration signal is recorded, and a time domain characteristic value of the vibration signal and/or a frequency domain characteristic value of the vibration signal may be further recorded, to obtain vibration data.

The acquisition time of the vibration data at least partially overlaps an acquisition time of the audio recording data. The vibration data includes, for example, data corresponding to all vibration signals from a moment at which a vibration signal triggers the microphone to be activated to a moment at which a last vibration signal is acquired before the audio recording data ends.

S302: The headset detects whether the audio recording data includes a cough sound.

Optionally, the headset may further detect, with reference to the vibration data, whether the audio recording data includes the cough sound. For example, based on a time when the vibration signal is detected, an audio recording segment within preset duration of the time corresponding to the audio recording data is obtained, and whether the audio recording segment includes the cough sound is detected. For another example, after detecting that the audio recording data includes the cough sound, the headset further determines whether data corresponding to the detected vibration signal exists in a time period corresponding to the cough sound in the vibration data. If data corresponding to the detected vibration signal exists in a time period corresponding to the cough sound in the vibration data, it may be determined that the audio recording data includes the cough sound. If data corresponding to the detected vibration signal does not exist in a time period corresponding to the cough sound in the vibration data, it may be determined that the cough sound is a false detection. If it is determined, based on the vibration data, that all cough sounds detected in the audio recording data are false detections, it may be determined that the audio recording data does not include the cough sound. Therefore, efficiency and accuracy of detecting the cough sound by the headset can be improved.

When the audio recording data includes the cough sound, S303 is performed. When the audio recording data does not include the cough sound, S304 is performed.

S303: The headset sends the audio recording data and the vibration data to a smart terminal device, so that the smart terminal device analyzes and collects statistics on cough information about the user based on the audio recording data and the vibration data.

Although not all vibration signals detected by the headset are caused by a cough of the user, the cough of the user is necessarily accompanied by vibration of a vocal cord and a skull of the user, so that corresponding vibration data can be recorded by the headset. Therefore, with reference to the vibration data, an acquired sound similar to a cough characteristic in an environment or an acquired cough sound of another person can be deleted from the audio recording data, thereby improving accuracy of detecting the cough sound.

Specifically, after detecting that the audio recording data includes the cough sound, the smart terminal device further determines whether the data corresponding to the detected vibration signal exists in the time period corresponding to the cough sound in the vibration data. If the data corresponding to the detected vibration signal exists in the time period corresponding to the cough sound in the vibration data, it may be determined that the cough sound is accurate, a quantity of coughs in the cough information is increased by 1, and a time corresponding to the cough sound is recorded. If the data corresponding to the detected vibration signal does not exist in the time period corresponding to the cough sound in the vibration data, it may be determined that the cough sound is a false detection, and the cough information is not updated temporarily.

Certainly, when the vibration data further includes the vibration characteristic value of the vibration signal, the smart terminal device may further determine, with reference to a vibration characteristic, whether the detected cough sound is accurate. For example, when the data corresponding to the detected vibration signal exists in the time period corresponding to the cough sound in the vibration data, and at least some values in the vibration characteristic value are greater than a threshold, it is determined that the cough sound is accurate. It may be understood that different vibration characteristic values correspond to different thresholds.

S304: The headset deletes the audio recording data and the vibration data.

When there is no cough sound in audio recording data, the audio recording data and the vibration data may be deleted to release storage space of the headset.

In this embodiment, the headset activates the microphone to perform audio recording only after detecting the vibration signal, so that a risk to user privacy security can be reduced, and power consumption of the headset can be further reduced. After detecting that the audio recording data includes the cough sound, the headset sends the audio recording data to the smart terminal device. Otherwise, the audio recording data and the vibration data are deleted. In this way, occupation of a storage resource of the headset can be reduced, and unnecessary data transmission between the headset and the smart terminal device is reduced, thereby reducing power consumption of the headset. Further, whether the audio recording data includes the cough sound is determined with reference to the vibration data, so that accuracy of recognizing the cough sound can be improved.

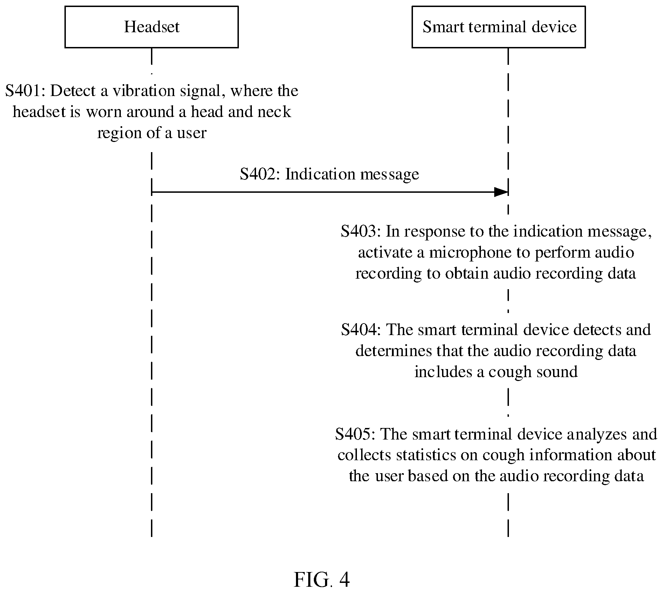

After detecting the vibration signal, the headset may not only activate the microphone of the headset to perform audio recording and detect whether the audio recording data includes the cough sound, as in the first embodiment and the second embodiment of the cough monitoring method, but also indicate the smart terminal device to activate the microphone to perform audio recording and detect the cough sound in the audio recording data without performing audio recording and detecting the cough sound by the headset itself. FIG. 4 is a schematic flowchart of a third embodiment of a cough monitoring method according to this application. This embodiment includes the following steps.

S401: A headset detects a vibration signal, where the headset is worn around a head and neck region of a user.

S402: In response to the vibration signal, the headset sends an indication message to a smart terminal device.

The indication message indicates the smart terminal device to activate a microphone to perform audio recording.

It may be understood that the headset does not send, to the smart terminal device, the indication message used to activate the microphone each time the headset detects the vibration signal. If a microphone of the smart terminal device is already activated when the headset detects the vibration signal, that is, before the headset indicates the smart terminal device to end audio recording, the headset does not need to send the indication message to the smart terminal device again.

S403: In response to the indication message, the smart terminal device activates the microphone to perform audio recording to obtain audio recording data.

After receiving the indication message, the smart terminal device activates the microphone to perform audio recording. A condition that the smart terminal device ends audio recording is as follows: For example, the smart terminal device receives a message that is sent by the headset and that indicates to end audio recording. The message that indicates to end audio recording is, for example, a message generated after the headset detects no vibration signal in a preset time period after the vibration signal is detected last time. The preset time period may be, for example, 30 seconds, 1 minute, 2 minutes, or 5 minutes. This is not limited herein.

S404: The smart terminal device detects and determines that the audio recording data includes a cough sound.

A method for detecting the cough sound by the smart terminal device is similar to S203, and therefore details are not described herein again.

S405: The smart terminal device analyzes and collects statistics on cough information about the user based on the audio recording data.

A method for collecting and analyzing the statistics on the cough information by the smart terminal device is similar to S203, and therefore details are not described herein again.

S404 and S405 may be implemented in a same step. To be specific, when detecting the cough sound in the audio recording data, the smart terminal device synchronously analyzes and collects the statistics on the cough information. For example, when a cough sound in audio recording data is detected, a quantity of coughs in cough information is increased by 1, and a time corresponding to the cough sound is recorded. If no cough sound is detected in the audio recording data after the audio recording data is detected, the cough information is not updated.

Optionally, after the smart terminal device completes detection of the audio recording data, the audio recording data may be deleted, thereby reducing storage space of the smart terminal device.

In this embodiment, the smart terminal device activates the microphone to perform audio recording only after receiving the indication message that is sent by the headset because the vibration signal is detected, which can reduce a risk to user privacy security, and also can reduce power consumption of the smart terminal device. In addition, the smart terminal device performs audio recording and detects the cough sound, which can reduce requirements for a storage resource and a computing resource of the headset, and reduce costs of the headset.

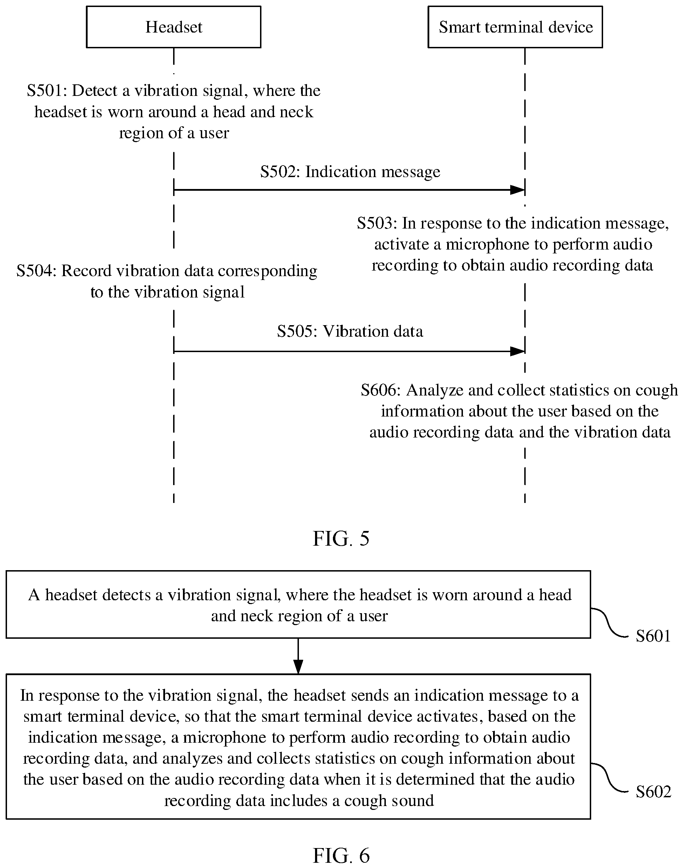

Similarly, the smart terminal may also detect and analyze and collect statistics on the cough sound in the audio recording data with reference to the vibration data acquired by the headset. FIG. 5 is a schematic flowchart of a fourth embodiment of a cough monitoring method according to this application. This embodiment includes the following steps.

S501: A headset detects a vibration signal, where the headset is worn around a head and neck region of a user.

S502: In response to the vibration signal, the headset sends an indication message to a smart terminal device.

The indication message indicates the smart terminal device to activate a microphone to perform audio recording.

S503: In response to the indication message, the smart terminal device activates the microphone to perform audio recording to obtain audio recording data.

After receiving the indication message, the smart terminal device activates the microphone to perform audio recording. A condition that the smart terminal device ends audio recording is as follows: For example, the smart terminal device receives a message that is sent by the headset and that indicates to end audio recording. The message that indicates to end audio recording is, for example, a message generated after the headset detects no vibration signal in a preset time period after the vibration signal is detected last time. The preset time period may be, for example, 30 seconds, 1 minute, 2 minutes, or 5 minutes. This is not limited herein.

S504: The headset records vibration data corresponding to the vibration signal.

The vibration data is data recorded when the headset detects the vibration signal. For the vibration data, refer to the related description in S301. Therefore, details are not described herein again.

It should be noted that, there is no sequence between S504 and S502, and S503, and recording the vibration data by the headset is a continuous process. All vibration signals detected during a period from a time when the headset detects the vibration signal used for activating the microphone of the smart terminal device to a time when the headset indicates the smart terminal device to end audio recording are recorded to obtain the vibration data.

S505: The headset sends the vibration data to the smart terminal device.

Both the vibration data and the message that indicates to end audio recording may be sent by the headset to the smart terminal device. Alternatively, the vibration data and the message that indicates to end audio recording may be separately sent by the headset to the smart terminal device. For example, the message that indicates to end audio recording may be first sent, and then the vibration data is sent. Alternatively, a reverse sending sequence may be used. This is not limited herein.

S506: The smart terminal device analyzes and collects statistics on cough information about the user based on the audio recording data and the vibration data.

A method for detecting the cough sound and analyzing and collecting the statistics on the cough information by the smart terminal device is similar to S203, and therefore details are not described herein again.

In this embodiment, the smart terminal device activates the microphone to perform audio recording only after receiving the indication message that is sent by the headset because the vibration signal is detected, which can reduce a risk to user privacy security, and also can reduce power consumption of the smart terminal device. In addition, the smart terminal device performs audio recording and detects the cough sound, which can reduce requirements for a storage resource and a computing resource of the headset, and reduce costs of the headset. Further, whether the audio recording data includes the cough sound is determined with reference to the vibration data, so that accuracy of recognizing the cough sound can be improved.

FIG. 6 is a schematic flowchart of a fifth embodiment of a cough monitoring method according to this application. This embodiment is executed by a headset. This embodiment includes the following steps.

S601: The headset detects a vibration signal, where the headset is worn around a head and neck region of a user.

S602: In response to the vibration signal, the headset sends an indication message to a smart terminal device, so that the smart terminal device activates, based on the indication message, a microphone to perform audio recording to obtain audio recording data, and analyzes and collects statistics on cough information about the user based on the audio recording data when it is determined that the audio recording data includes a cough sound.

For a related operation of the headset in this embodiment, refer to related content of the third embodiment of the cough monitoring method or the fourth embodiment of the cough monitoring method. Details are not described herein again.

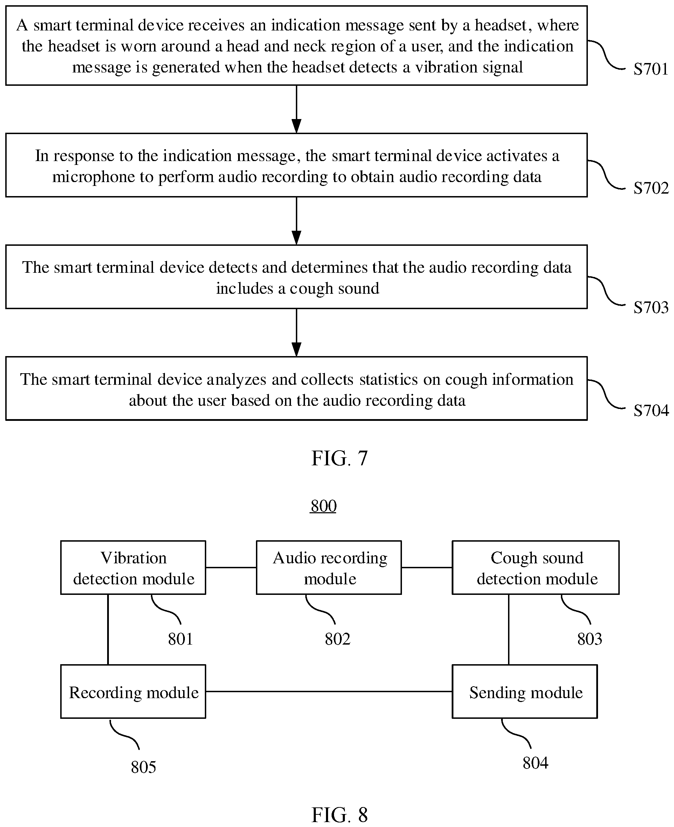

FIG. 7 is a schematic flowchart of a sixth embodiment of a cough monitoring method according to this application. This embodiment is executed by a smart terminal device. This embodiment includes the following steps.

S701: The smart terminal device receives an indication message sent by a headset, where the headset is worn around a head and neck region of a user, and the indication message is generated when the headset detects a vibration signal.

S702: In response to the indication message, the smart terminal device activates a microphone to perform audio recording to obtain audio recording data.

S703: The smart terminal device detects and determines that the audio recording data includes a cough sound.

S704: The smart terminal device analyzes and collects statistics on cough information about the user based on the audio recording data.

For a related operation of the smart terminal device in this embodiment, refer to related content of the third embodiment of the cough monitoring method or the fourth embodiment of the cough monitoring method. Details are not described herein again.

The first embodiment or the second embodiment of the cough monitoring method is implemented by a headset 800. FIG. 8 is a diagram of a structure of an embodiment of a headset according to this application. The headset 800 includes:
a vibration detection module 801, configured to detect a vibration signal;
an audio recording module 802, configured to: when the vibration detection module 801 detects the vibration signal, activate a microphone to perform audio recording to obtain audio recording data;
a cough sound detection module 803, configured to detect whether the audio recording data includes a cough sound; and
a sending module 804, configured to send the audio recording data to a smart terminal device when it is determined that the audio recording data includes the cough sound, so that the smart terminal device analyzes and collects statistics on cough information about a user based on the audio recording data.

In a possible implementation, the cough sound detection module 803 is specifically configured to: compare a similarity between the audio recording data and cough sound data, where the cough sound data is previously recorded cough sound data of the user; and when the similarity between the audio recording data and the cough sound data is greater than or equal to a threshold, determine that the audio recording data includes the cough sound; or when the similarity between the audio recording data and the cough sound data is less than the threshold, determine that the audio recording data does not include the cough sound.

In a possible implementation, the headset further includes: a recording module 805, configured to record vibration data corresponding to the detected vibration signal. The sending module 804 is further configured to send the vibration data to the smart terminal device, so that the smart terminal device analyzes and collects the statistics on the cough information based on the audio recording data and the vibration data.

In a possible implementation, the audio recording data includes audio data and an acquisition time of the audio data, and the cough information includes at least one of a cough time, a quantity of coughs, a cough frequency, a cough trend, and cough duration.

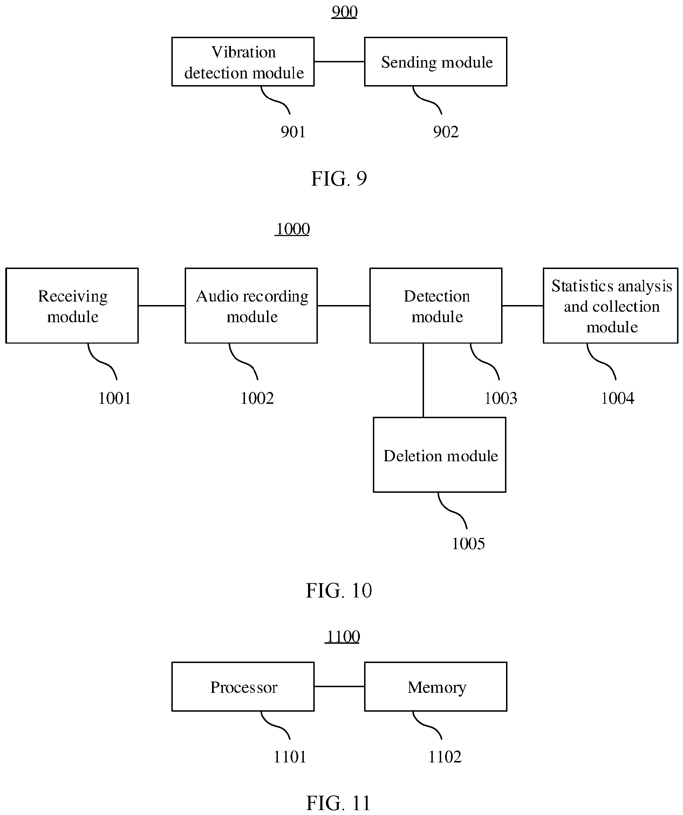

An execution action of the headset in the third embodiment, the fourth embodiment, or the fifth embodiment of the cough monitoring method is implemented by a headset 900. FIG. 9 is a diagram of a structure of another embodiment of a headset according to this application. The headset 900 includes: a vibration detection module 901, configured to detect a vibration signal; and a sending module 902, configured to send an indication message to a smart terminal device when the vibration signal is detected, so that the smart terminal device activates, based on the indication message, a microphone to perform audio recording to obtain audio recording data, and analyzes and collects statistics on cough information about a user based on the audio recording data when it is determined that the audio recording data includes a cough sound.

In a possible implementation, the sending module 902 is further configured to send vibration data to the smart terminal device, so that the smart terminal device analyzes and collects the statistics on the cough information about the user based on the audio recording data and the vibration data. The vibration data is corresponding data recorded when the vibration detection module 901 detects the vibration signal.

An execution action of the smart terminal device in the third embodiment, the fourth embodiment, or the fifth embodiment of the cough monitoring method is implemented by a cough detection apparatus 1000. FIG. 10 is a diagram of a structure of an embodiment of a cough monitoring apparatus according to this application. The apparatus 1000 includes:
a receiving module 1001, configured to receive an indication message sent by a headset, where the headset is worn around a head and neck region of a user, and the indication message is generated when the headset detects a vibration signal;
an audio recording module 1002, configured to: when the indication message is received, activate a microphone to perform audio recording to obtain audio recording data;
a detection module 1003, configured to detect and determine that the audio recording data includes a cough sound; and
a statistics analysis and collection module 1004, configured to analyze and collect statistics on cough information about the user based on the audio recording data.

In a possible implementation, the detection module 1003 is specifically configured to: compare a similarity between the audio recording data and cough sound data, where the cough sound data is previously recorded cough sound data of the user; and when it is determined that the similarity between the audio recording data and the cough sound data is greater than or equal to a threshold, determine that the audio recording data includes the cough sound.

In a possible implementation, the receiving module 1001 is further configured to receive vibration data sent by the headset, where the vibration data is data that corresponds to the detected vibration signal and that is recorded by the headset. The statistics analysis and collection module 1004 is configured to analyze and collect the statistics on the cough information based on the audio recording data and the vibration data.

In a possible implementation, the apparatus 1000 includes a deletion module 1005, configured to delete the audio recording data when the audio recording data does not include the cough sound.

FIG. 11 is a diagram of a structure of an embodiment of a cough monitoring device according to this application. The cough detection device 1100 includes a processor 1101 and a memory 1102. The processor 1101 is coupled to the memory 1102, and the processor 1101 is configured to perform, based on instructions stored in the memory 1102, the cough monitoring method performed by the headset in any one of FIG. 2 to FIG. 6, or the cough monitoring method performed by the smart terminal device in FIG. 3, FIG. 4, or FIG. 7.

This application further provides a computer-readable storage medium, and the computer-readable storage medium stores a computer program. When the computer program is executed by a computer, the procedure of the cough monitoring method in any one of the foregoing method embodiments is implemented.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In the several embodiments provided in this application, it should be understood that the disclosed system, apparatus, and method may be implemented in other manners. For example, the described apparatus embodiment is merely an example. For example, division into the units is merely logical function division and may be other division during actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic or other forms.

The units described as separate parts may be or may not be physically separate, and parts displayed as units may be or may not be physical units, may be located in one position, or may be distributed on a plurality of network units. Some or all of the units may be selected based on actual requirements to achieve objectives of solutions of embodiments.

In addition, functional units in embodiments of this application may be integrated into one processing unit, or each of the units may exist alone physically, or two or more units may be integrated into one unit. The integrated unit may be implemented in a form of hardware, or may be implemented in a form of a software functional unit.

When the integrated unit is implemented in the form of the software functional unit and sold or used as an independent product, the integrated unit may be stored in a computer-readable storage medium. Based on such an understanding, all or a part of the technical solutions of this application may be implemented in a form of a software product. The computer software product is stored in a storage medium, and includes several instructions for enabling a computer device (which may be a personal computer, a server, or a network device) to perform all or some of the steps of the methods in embodiments of this application. The storage medium includes any medium that can store program code, such as a USB flash drive, a removable hard disk, a read-only memory (ROM, read-only memory), a random access memory (RAM, random access memory), a magnetic disk, or an optical disc.

## Claims

1. A cough monitoring method, wherein the method comprises:
in response to a detected vibration signal, activating, by a headset, a microphone to perform audio recording to obtain audio recording data, wherein the headset is worn around a head and neck region of a user;
detecting, by the headset, whether the audio recording data comprises a cough sound; and
if the audio recording data comprises the cough sound, sending, by the headset, the audio recording data to a smart terminal device, so that the smart terminal device analyzes and collects statistics on cough information about the user based on the audio recording data.

2. The method according to claim 1, wherein the detecting, by the headset, whether the audio recording data comprises a cough sound comprises:
comparing, by the headset, a similarity between the audio recording data and cough sound data, wherein the cough sound data is previously recorded cough sound data of the user; and
if the similarity between the audio recording data and the cough sound data is greater than or equal to a threshold, determining, by the headset, that the audio recording data comprises the cough sound; or
if the similarity between the audio recording data and the cough sound data is less than the threshold, determining, by the headset, that the audio recording data does not comprise the cough sound.

3. The method according to claim 1 or 2, wherein the method further comprises:
recording, by the headset, vibration data corresponding to the detected vibration signal; and
the sending, by the headset, the audio recording data to a smart terminal device, so that the smart terminal device analyzes and collects statistics on cough information based on the audio recording data comprises:
sending, by the headset, the audio recording data and the vibration data to the smart terminal device, so that the smart terminal device analyzes and collects the statistics on the cough information based on the audio recording data and the vibration data.

4. The method according to any one of claims 1 to 3, wherein the audio recording data comprises audio data and an acquisition time of the audio data, and the cough information comprises at least one of a cough time, a quantity of coughs, a cough frequency, a cough trend, and cough duration.

5. The method according to any one of claims 1 to 4, wherein the method further comprises:
if the audio recording data does not comprise the cough sound, deleting, by the headset, the audio recording data.

6. A cough monitoring method, wherein the method comprises:
detecting, by a headset, a vibration signal, wherein the headset is worn around a head and neck region of a user; and
in response to the vibration signal, sending, by the headset, an indication message to a smart terminal device, so that the smart terminal device activates, based on the indication message, a microphone to perform audio recording to obtain audio recording data, and analyzes and collects statistics on cough information about the user based on the audio recording data when it is determined that the audio recording data comprises a cough sound.

7. A cough monitoring method, wherein the method comprises:
receiving, by a smart terminal device, an indication message sent by a headset, wherein the headset is worn around a head and neck region of a user, and the indication message is generated when the headset detects a vibration signal;
in response to the indication message, activating, by the smart terminal device, a microphone to perform audio recording to obtain audio recording data;
detecting and determining, by the smart terminal device, that the audio recording data comprises a cough sound; and
analyzing and collecting, by the smart terminal device, statistics on cough information about the user based on the audio recording data.

8. The method according to claim 7, wherein the detecting and determining, by the smart terminal device, that the audio recording data comprises a cough sound comprises:
comparing, by the smart terminal device, a similarity between the audio recording data and cough sound data, wherein the cough sound data is previously recorded cough sound data of the user; and
when the smart terminal device determines that the similarity between the audio recording data and the cough sound data is greater than or equal to a threshold, determining, by the smart terminal device, that the audio recording data comprises the cough sound.

9. The method according to claim 7 or 8, wherein the method further comprises:
receiving, by the smart terminal device, vibration data sent by the headset, wherein the vibration data is data that corresponds to the detected vibration signal and that is recorded by the headset; and
the analyzing and collecting, by the smart terminal device, statistics on cough information based on the audio recording data comprises:
analyzing and collecting, by the smart terminal device, the statistics on the cough information based on the audio recording data and the vibration data.

10. The method according to any one of claims 7 to 9, wherein the audio recording data comprises audio data and an acquisition time of the audio data, and the cough information comprises at least one of a cough time, a quantity of coughs, a cough frequency, a cough trend, and cough duration.

11. A cough monitoring method, wherein the method comprises:
detecting, by a headset, a vibration signal, wherein the headset is worn around a head and neck region of a user;
in response to the vibration signal, sending, by the headset, an indication message to a smart terminal device;
in response to the indication message, activating, by the smart terminal device, a microphone to perform audio recording to obtain audio recording data;
detecting and determining, by the smart terminal device, that the audio recording data comprises a cough sound; and
analyzing and collecting, by the smart terminal device, statistics on cough information about the user based on the audio recording data.

12. A cough monitoring system, wherein the system comprises a headset and a smart terminal device, wherein
the headset is worn around a head and neck region of a user, and is configured to detect a vibration signal;
the headset is further configured to: when detecting the vibration signal, activate a microphone to perform audio recording to obtain audio recording data;
the headset is further configured to detect whether the audio recording data comprises a cough sound;
the headset is further configured to send the audio recording data to the smart terminal device when the audio recording data comprises the cough sound; and
the smart terminal device is configured to analyze and collect statistics on cough information about the user based on the audio recording data.

13. A cough monitoring system, wherein the system comprises a headset and a smart terminal device, wherein
the headset is worn around a head and neck region of a user, and is configured to detect a vibration signal;
the headset is configured to send an indication message to the smart terminal device when the vibration signal is detected;
the smart terminal device is configured to activate, based on the indication message, a microphone to perform audio recording to obtain audio recording data; and
the smart terminal device is further configured to analyze and collect statistics on cough information about the user based on the audio recording data when it is detected and determined that the audio recording data comprises a cough sound.

14. A headset, wherein the headset comprises:
a vibration detection module, configured to detect a vibration signal;
an audio recording module, configured to: when the vibration detection module detects the vibration signal, activate a microphone to perform audio recording to obtain audio recording data;
a cough sound detection module, configured to detect whether the audio recording data comprises a cough sound; and
a sending module, configured to send the audio recording data to a smart terminal device when it is determined that the audio recording data comprises the cough sound, so that the smart terminal device analyzes and collects statistics on cough information about the user based on the audio recording data.

15. The headset according to claim 14, wherein the cough sound detection module is specifically configured to:
compare a similarity between the audio recording data and cough sound data, wherein the cough sound data is previously recorded cough sound data of the user; and
when the similarity between the audio recording data and the cough sound data is greater than or equal to a threshold, determine that the audio recording data comprises the cough sound; or when the similarity between the audio recording data and the cough sound data is less than the threshold, determine that the audio recording data does not comprise the cough sound.

16. The headset according to claim 14 or 15, wherein the headset further comprises:
a recording module, configured to record vibration data corresponding to the detected vibration signal, wherein
the sending module is further configured to send the vibration data to the smart terminal device, so that the smart terminal device analyzes and collects the statistics on the cough information based on the audio recording data and the vibration data.

17. The headset according to any one of claims 14 to 16, wherein the audio recording data comprises audio data and an acquisition time of the audio data, and the cough information comprises at least one of a cough time, a quantity of coughs, a cough frequency, a cough trend, and cough duration.

18. A headset, wherein the headset comprises:
a vibration detection module, configured to detect a vibration signal; and
a sending module, configured to send an indication message to a smart terminal device when the vibration signal is detected, so that the smart terminal device activates, based on the indication message, a microphone to perform audio recording to obtain audio recording data, and analyzes and collects statistics on cough information about the user based on the audio recording data when it is determined that the audio recording data comprises a cough sound.

19. A cough monitoring apparatus, wherein the apparatus comprises:
a receiving module, configured to receive an indication message sent by a headset, wherein the headset is worn around a head and neck region of a user, and the indication message is generated when the headset detects a vibration signal;
an audio recording module, configured to: when the indication message is received, activate a microphone to perform audio recording to obtain audio recording data;
a detection module, configured to detect and determine that the audio recording data comprises a cough sound; and
a statistics analysis and collection module, configured to analyze and collect statistics on cough information about the user based on the audio recording data.

20. A cough monitoring device, wherein the device comprises a processor and a memory, the processor is coupled to the memory, and the processor is configured to perform, based on instructions stored in the memory, the cough monitoring method according to any one of claims 1 to 10.

21. A computer-readable storage medium, comprising instructions, wherein when the computer-readable storage medium is run on a computer, the computer is enabled to perform the cough monitoring method according to any one of claims 1 to 10.
